# EUROPEAN PATENT APPLICATION

(11) **EP 3 573 070 A1**
(43) Date of publication of application: **27.11.2019**
(21) Application number: 18173478.1
(22) Date of filing: 22.05.2018
(51) Int. Cl.: G16H 40/63, A61B 5/145, A61B 5/00

(54) **HANDS DISPLAY OF SMARTWATCH FOR GLUCOSE MONITORING**

(71) Applicant: Manufacture Modules Technologies SA, 1228 Plan-les-Ouates (CH)
(72) Inventor: FRABOULET, Philippe, 1228 Plan-les-Ouates (CH)
(74) Representative: Weihs, Bruno Konrad

(57) **Abstract**

A smartwatch is configured for continuous glucose monitoring in the blood of an intended user using the smartwatch, the smartwatch comprising an analog display with a large hand and a small hand which are configured to show the time in a conventional manner; a control means configured to adjust the time by causing the large hand and the small hand to rotate when the control means is actuated by the intended user; a radio receiver enabled to receive radio waves that carry measurements of glucose and extract the measurements of glucose; a processing system configured to input the measurements of glucose from the radio receiver and further to control positions of the large hand and the small hand as a function of the processed measurements of glucose, whereby the large hand is dedicated to display values of the processed measurements of glucose on an outer scale of the analog display and the small hand is dedicated to point to signs printed on the analog display indicating either one of a tendency of the values of the processed measurements to increase or a tendency of the processed measurements to fall over time.

## Description

### Field of invention

The invention is in the field of continuous monitoring of glucose measurements.s

### Background of the invention

Systems for continuous glucose measurement in patients suffering from diabetes allow to adapt the administering of insulin in real time, for example manually. Such system may rely on an implantable glucose measuring device implanted on a user, e.g., on the skin of the user, and that comprises a radio transmitter, whereby the result of the measurement is transmitted by radio signal to a receiver, which in turn is either itself enabled to evaluate and display the result to the user, or alternatively outputs the results to a dedicated device that is enabled to evaluate and display the result to the user.

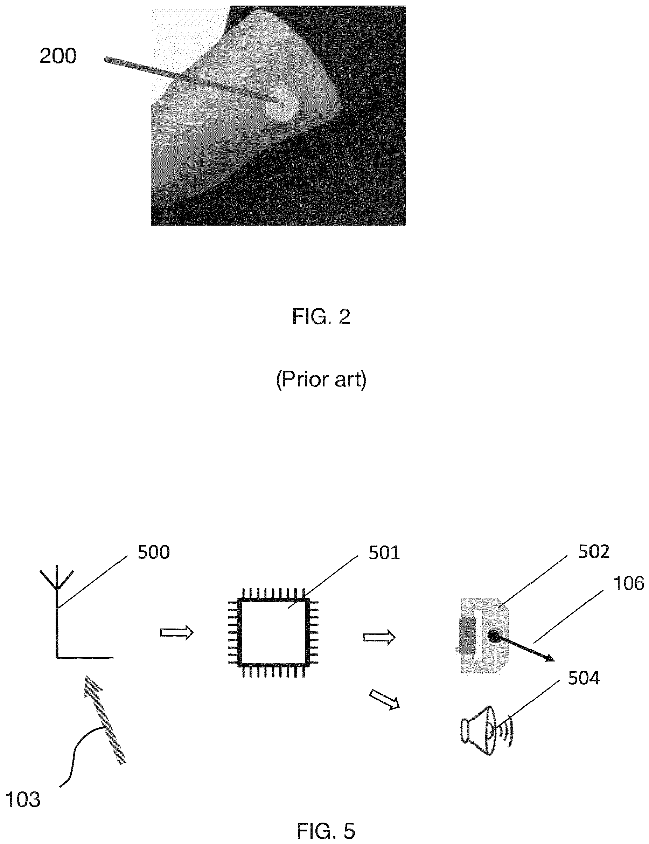

International publication WO 2018/049167 discloses a prior art example of a system for continuous glucose measurement. The system may comprise a continuous analyte sensor, which includes an embodiment of a glucose sensor. Such an analyte sensor 200 is shown in the example of figure 2, where it is implanted on the skin of a user's upper arm in a known fashion. The system makes use of a communication medium, e.g., a wireless communication according to the BLE standard (Bluetooth® Low Energy) to couple the analyte sensor to a display device. To this end, the analyte sensor 200 may comprise a radio emitter. The system is enabled to produce new measurements that may be transmitted often enough to adequately monitor glucose levels, whereby the time interval between measurements transmitted can be varied to be any desired length of time. The display device may be a smart watch, which displays numerical values associated with analyte data received.

US patent publication US 2008/0068932 concerns a wristwatch for monitoring glucose of a diabetic user. The monitoring is said to be continuous, and correlated with other measurements to alert the user in case certain thresholds are reached. The wristwatch is of the analog type with the usual large hands showing the time, and dedicated smaller hands located below the large hands, and each having one hand pointing to a value representative of the respective measurement, i.e., one of the smaller hands indicating the glucose level.

The present invention aims at improving the systems from prior art.

### Summary of invention

In a first aspect, the invention provides a smartwatch configured for continuous glucose monitoring in the blood of an intended user using the smartwatch, the smartwatch comprising an analog display with a large hand and a small hand which are configured to show the time in a conventional manner; a control means configured to adjust the time by causing the large hand and the small hand to rotate when the control means is actuated by the intended user; a radio receiver enabled to receive radio waves that carry measurements of glucose and extract the measurements of glucose; a processing system configured to input the measurements of glucose from the radio receiver and further to control positions of the large hand and the small hand as a function of the processed measurements of glucose, whereby the large hand is dedicated to display values of the processed measurements of glucose on an outer scale of the analog display and the small hand is dedicated to point to signs printed on the analog display indicating either one of a tendency of the values of the processed measurements to increase or a tendency of the processed measurements to fall over time.

In a preferred embodiment, the control means is further configured to trigger a change of display mode between a first mode wherein the large hand is dedicated to display values of the processed measurement of glucose on the outer scale and the small hand is dedicated to point to signs printed on the analog display indicating either one of the tendency to increase of the tendency to fall over time, and a second mode wherein the large hand and the small hand are dedicated to show the time in a conventional manner on the analog display.

In a further preferred embodiment, the smartwatch further comprises radio emitting means which are enabled to communicate through radio waves in order to transmit the processed measurements of glucose to an intended computing device distinct from the smartwatch.

In a further preferred embodiment, the smartwatch is further configured to store a pre-determined threshold value, to compare the processed measurement values with the threshold value, and to trigger an alarm when a processed measurement value reaches the pre-determined threshold value.

In a second aspect, the invention provides a continuous glucose measurement system comprising a glucose measurement device, a transmitter device, the glucose measurement being connected to the transmitter device, which is configured to input the measurements of the glucose level from the glucose measurement device, and output the measurements of the glucose level by means of radio waves, a computing device, a smartwatch configured for continuous glucose monitoring in the blood of an intended user using the smartwatch, the smartwatch comprising an analog display with a large hand and a small hand which are configured to show the time in a conventional manner, a control means configured to adjust the time by causing the large hand and the small hand to rotate when the control means is actuated by the intended user, a radio receiver enabled to receive the radio waves from the transmitter device, that carry measurements of glucose and extract the measurements of glucose, a processing system configured to input the measurements of glucose from the radio receiver and further to control positions of the large hand and the small hand as a function of the processed measurements of glucose, whereby the large hand is dedicated to display values of the processed measurements of glucose on an outer scale of the analog display and the small hand is dedicated to point to signs printed on the analog display indicating either one of a tendency of the values of the processed measurements to increase or a tendency of the processed measurements to fall over time, a radio emitting means enabled to communicate through radio waves in order to transmit the glucose measurement values, wherein the computing device is configured to receive the glucose measurement values received from the radio emitting means.

In a further preferred embodiment, the computing is further configured to execute a smartwatch companion application, enabling to configure the smartwatch with parameters.

In a further preferred embodiment, the companion application enables an intended user to set on the smartwatch a particular a pre-determined threshold or conditions for the glucose measurement, the smartwatch being enabled to detect when the pre-determined threshold is reached or the conditions are met, on the glucose measurement values received in real time by the smartwatch from the glucose measurement device, and to trigger an alert for the user.

In a further preferred embodiment, the measurement device comprises:
- a biosensor implanted in or on the skin of the intended user,
- an electronic module reading the value of the biosensor.

### Brief description of the drawings

The invention will be better understood through the description of preferred example embodiments and in reference to the figures wherein
figure 1 shows an example embodiment of a smartwatch embedded in a continuous glucose monitoring system, according to the invention;
figure 2 shows an example analyte sensor, in this case a glucose measuring device, implanted on the surface of a user's upper arm skin according to prior art;
figure 3 shows an analogue display with hands pointing at values according to a preferred embodiments of the invention;
figure 4 shows an analogue display with hands pointing at values according to a further preferred embodiment of the invention; and
figure 5 shows functional blocks of a smartwatch.

### Detailed description of preferred example embodiments of the invention

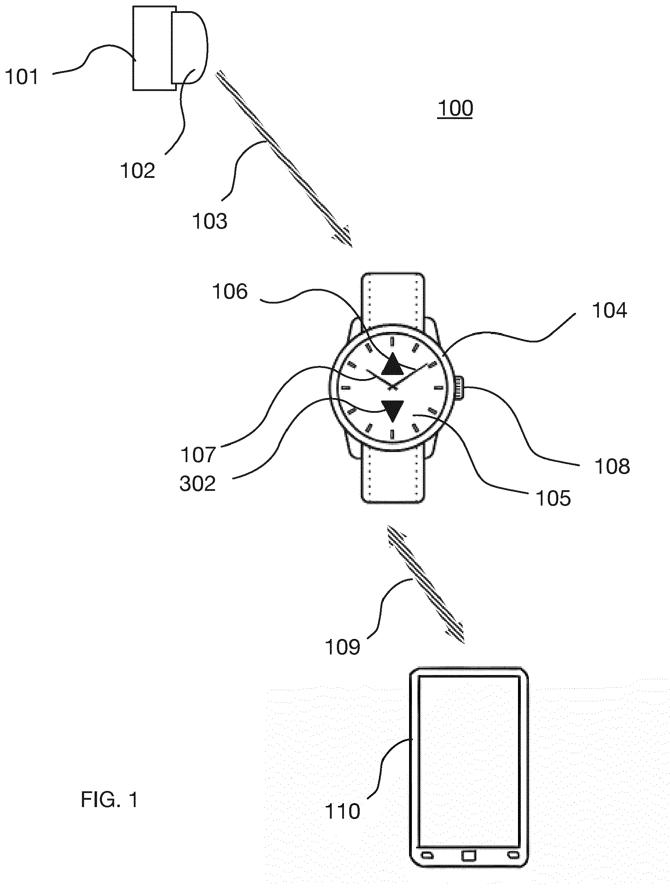

Figure 1 is a schematic representation of a continuous glucose monitoring (CGM) system 100. The system comprise a glucose measurement device 101 configured to be implanted for example on the skin of a user (both skin and user not represented in figure 1, but one example is shown in figure 2) and make measurements of a glucose level in the blood of the user at a predetermined frequency. The glucose measurement device 101 is connected to a transmitter device 102, which is configured to input the measurements of the glucose level from the glucose measurement device 101, and output the measurements of the glucose level by means of radio waves as symbolized by arrow 103, for example according to the Bluetooth® Low Energy standard.

The CGM system 100 further comprises a smartwatch 104, according to an example embodiment of the invention, the smartwatch 104 comprising a conventional analog display 105 with a large hand 106 and a small hand 107 which may, in a display mode, show the time in a usual matter. The smartwatch 104 further comprises a control means 108, for example a pusher button, or a plurality of pusher buttons, or a rotatable crown, which may be used to simulate or implement in a known manner a means for adjusting the time by causing the large hand 106 and the small hand 107 to rotate when actuated. Of course, the smartwatch may comprise more than a single control means 108, however this is not illustrated in figure 1 for a better readability. The smartwatch 104 further comprises a radio receiver 500, as shown in figure 5 where functional blocks of the smartwatch are schematically illustrated, the radio receiver being enabled to receive the radio waves 103 and extract the measurements of the glucose level. The smartwatch 104 further comprises a processing system 501 configured to input the measurements of the glucose level from the radio receiver 500, and further to control positions of the large hand 106 and the small hand 107, e.g., to drive Lavet-type motors 502 to control positions of the hands. In Figure 5 is represented one Lavet-type motor 502 controlling the position of one hand 106. In particular the processing system may process the input measurements of the glucose level and output settings for the large hand 106 and the small hand 107, that may displayed by appropriate movement of the large hand 106 and the small hand 107 in a further display mode. This may be in a continuous display or alternatively, after this has been requested by the user by actuating the crown 108.

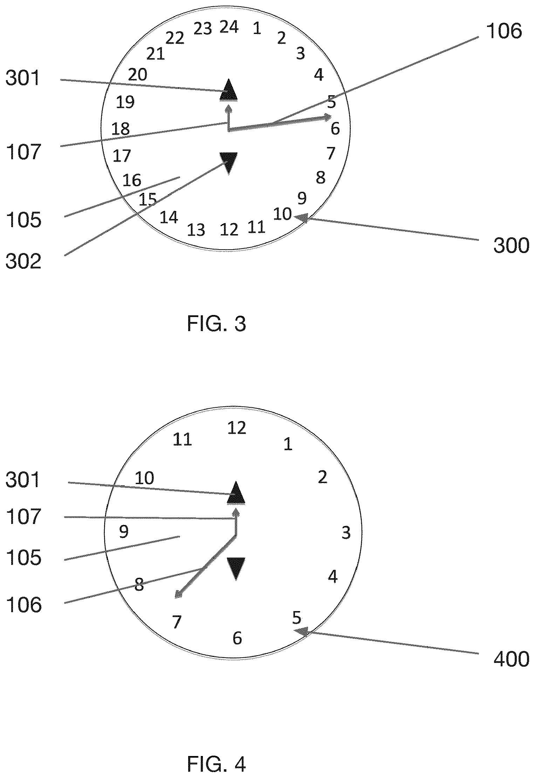

In a preferred embodiment, as illustrated in figure 3, the large hand 106 points to a 24-hours scale 300 of the analog display 105 with large numbers on the outer scale so the user may easily read on the outer scale. In the example of figure 3, the large hand 106 points between the large numbers 5 and 6, indicating an intermediate value. In a further preferred embodiment, on the minute track, i.e., between the large numbers on the outer scale, there is printed a one fourth division so measurement digits may be seen. This is not illustrated in figure 3. This allows to display decimal values of glucose measurement of the glucose level. The small hand 107 points to an "UP" triangle 301 to display a tendency of rising of the glucose measurements values over a period of time, thereby showing a trend in the processed measurements of the glucose over a determined period of time. In a similar manner, the small hand 107 may point to a "DOWN" triangle 302 to display falling glucose measurement values. When the measurement trend is stable the small hand shows a flat symbol (not shown in figure 3) or is oriented in a horizontal direction extending between the large numbers 6 and 18.

In a further preferred embodiment, as illustrated in figure 4, the large hand 106 points to a 12-hours scale 400 of the analogue display 105 with large numbers on the outer scale so the user may easily read on the outer scale. In the example of figure 4, the large hand 106 points between the large numbers 7 and 8, indicating an intermediate value. The small hand 107 points to the "UP" triangle 301 to display a tendency of rising of the glucose measurements values over a period of time, thereby showing a trend in the processed measurements of the glucose over a determined period of time.

Returning to figure 1, the smartwatch 104 according to the invention may comprise a radio emitting means (not shown in figure 1) which is enabled to communicate through radio waves 109 in order to transmit the glucose measurement values (not illustrated in figure 1) to a computing device 110, e.g., a smartphone, for storage and/or further processing.

In a preferred embodiment, the radio receiver of the smartwatch may be enable to also receive radio signals from the computing device 110. This may be useful, for example, to run a companion application om the computing device 110, which interacts with the smartwatch to parameter the smartwatch.

In a preferred embodiment, the smartwatch may be configured by the computing device 110, to enable alerts when pre-defined thresholds of the glucose measurement value are reached. The threshold levels for minimum and maximum glucose levels are set by the computing device 110 to the processing system 501 of the smartwatch 104. The processing system 501 continuously receives via the radio receiver 500, the values of the CGM system 100. The processing system 501 compares the received values with the values set by the computing device 110. When the received value is above maximum or under minimum, the processing system 501 generates an alert by sending a notification to the computing system 110 and driving the sound speaker 504.

In a preferred embodiment, the alert is silently displayed by the smartwatch 104 by turning one or two hands continuously in one direction. The hands remain turning until the user stops the alert by pressing the crown 108. The smartwatch 104 display then goes back to previous display mode.

In a further preferred embodiment, the smartwatch may be configured such that when the hands are used to display the measurement of the glucose level, then if the smartwatch is not connected to the measurement device, the small hand shows a "Not Connected" symbol or text, to let the user know that there is not any valid glucose measurement sent to the smartwatch.

In summary, when the large and small hands are used to display measurement of the glucose level, the comparatively long large hand 106 is pointing to comparatively large numerals on the outer scale of the analog smartwatch, which makes readings significantly more readable than in prior art. In addition, the applicant has found that reading time and measurement values via analog hands is preferred by many users as compared to digital number displays.

As a further advantage of the present invention, the read-out of measurements via analog hands enables traditional watch designs and stimulates users to continue to wear these wristwatches.

In one preferred embodiment, a press on the crown 108 pusher of the smartwatch may be configured to have the center hands to time display where the large hand 106 displays minutes and the small hand 107 displays hours. Three seconds after the crown 108 pusher activation, the large and small hands return to the glucose measurement display. The smartwatch is enabled such that the user is able to change the settings numbers of seconds that time is displayed after the crown 108 push.

## Claims

1. A smartwatch configured for continuous glucose monitoring in the blood of an intended user using the smartwatch, the smartwatch (104) comprising an analog display (105) with a large hand (106) and a small hand (107) which are configured to show the time in a conventional manner;
a control means (108) configured to adjust the time by causing the large hand (106) and the small hand (107) to rotate when the control means is actuated by the intended user;
a radio receiver (500) enabled to receive radio waves that carry measurements of glucose and extract the measurements of glucose;
a processing system (501) configured to input the measurements of glucose from the radio receiver (500) and further to control positions (502) of the large hand (106) and the small hand (107) as a function of the processed measurements of glucose, whereby the large hand (106) is dedicated to display values of the processed measurements of glucose on an outer scale of the analog display and the small hand (107) is dedicated to point to signs printed on the analog display indicating either one of a tendency of the values of the processed measurements to increase or a tendency of the processed measurements to fall over time.

2. The smartwatch of claim 1, in which the control means (108) is further configured to trigger a change of display mode between a first mode wherein the large hand is dedicated to display values of the processed measurement of glucose on the outer scale and the small hand is dedicated to point to signs printed on the analog display indicating either one of the tendency to increase of the tendency to fall over time, and a second mode wherein the large hand and the small hand are dedicated to show the time in a conventional manner on the analog display.

3. The smartwatch of claim 1, further comprising radio emitting means which are enabled to communicate through radio waves in order to transmit the processed measurements of glucose to an intended computing device distinct from the smartwatch.

4. The smartwatch of claim 1 further configured to store a pre-determined threshold value, to compare the processed measurement values with the threshold value, and to trigger an alarm when a processed measurement value reaches the pre-determined threshold value.

5. A continuous glucose measurement system comprising
a glucose measurement device (101),
a transmitter device (102),
the glucose measurement being connected to the transmitter device (102), which is configured to input the measurements of the glucose level from the glucose measurement device 101, and output the measurements of the glucose level by means of radio waves (103),
a computing device (110),
a smartwatch configured for continuous glucose monitoring in the blood of an intended user using the smartwatch, the smartwatch (104) comprising
an analog display (105) with a large hand 106 and a small hand (107) which are configured to show the time in a conventional manner,
a control means (108) configured to adjust the time by causing the large hand (106) and the small hand (107) to rotate when the control means is actuated by the intended user,
a radio receiver (500) enabled to receive the radio waves from the transmitter device, that carry measurements of glucose and extract the measurements of glucose,
a processing system (501) configured to input the measurements of glucose from the radio receiver and further to control positions (502) of the large hand (106) and the small hand (107) as a function of the processed measurements of glucose, whereby the large hand (106) is dedicated to display values of the processed measurements of glucose on an outer scale of the analog display and the small hand (107) is dedicated to point to signs printed on the analog display indicating either one of a tendency of the values of the processed measurements to increase or a tendency of the processed measurements to fall over time,
a radio emitting means enabled to communicate through radio waves in order to transmit the glucose measurement values,
wherein the computing device (110) is configured to receive the glucose measurement values received from the radio emitting means.

6. The continuous glucose measurement system of claim 5,
wherein the computing is further configured to execute a smartwatch companion application, enabling to configure the smartwatch with parameters.

7. The continuous glucose measurement system of claim 6, wherein the companion application enables an intended user to set on the smartwatch a particular a pre-determined threshold or conditions for the glucose measurement, the smartwatch being enabled to detect when the pre-determined threshold is reached or the conditions are met, on the glucose measurement values received in real time by the smartwatch from the glucose measurement device, and to trigger an alert for the user.

8. The continuous glucose measurement system of claim 5, in which the measurement device comprises:
- a biosensor implanted in or on the skin of the intended user,
- an electronic module reading the value of the biosensor.
